# EUROPÄISCHE PATENTSCHRIFT

(11) **EP 3 502 083 B1**
(45) Veröffentlichungstag und Bekanntmachung des Hinweises auf die Patenterteilung: **01.07.2020**
(21) Anmeldenummer: 17209329.6
(22) Anmeldetag: 21.12.2017
(51) Int. Cl.: C07C 51/14, C07C 53/126

(54) **VERFAHREN ZUR PD-KATALYSIERTEN HYDROXYCARBONYLIERUNG VON DIISOBUTEN: VERHÄLTNIS LIGAND/PD**
METHOD FOR THE PD CATALYTIC HYDROXYCARBONYLATION OF DI-ISOBUTENE: PD/LIGAND RATIO
PROCÉDÉ D'HYDROXYCARBONYLATION DE DIISOBUTÈNE CATALYSÉE PAR LE PALLADIUM : RAPPORT ENTRE LE LIGAND ET LE PD

(43) Veröffentlichungstag der Anmeldung: 26.06.2019
(73) Patentinhaber: Evonik Operations GmbH, 45128 Essen (DE)
(72) Erfinder: SANG, Rui, 252000 Liaocheng City, Shandong Province (CN); KUCMIERCZYK, Peter, 44652 Herne (DE); DONG, Kaiwu, 236800 Bo Zhou (CN); JACKSTELL, Ralf, 10106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- EP-A1- 1 657 230
- ANNE BRENNFÜHRER ET AL: "Palladium-Catalyzed Carbonylation Reactions of Alkenes and Alkynes", CHEMCATCHEM, Bd. 1, Nr. 1, 24. August 2009 (2009-08-24) , Seiten 28-41, XP055121332, ISSN: 1867-3880, DOI: 10.1002/cctc.200900062

## Beschreibung

Die Erfindung betrifft ein Verfahren zur Pd-katalysierten Hydroxycarbonylierung von Diisobuten: Verhältnis Ligand/Pd.

Carbonsäuren einschließlich Propionsäure, Adipinsäure und Fettsäuren werden bei der Herstellung von Polymeren, Pharmazeutika, Lösungsmitteln und Lebensmittelzusatzstoffen verwendet. Zu den Routen, welche zu Carbonsäuren führen, gehören im Allgemeinen die Oxidation von Kohlenwasserstoffen, Alkoholen oder Aldehyden, die oxidative Spaltung von Olefinen durch Ozonolyse, die Hydrolyse von Triglyceriden, Nitrilen, Estern oder Amiden, die Carboxylierung von Grignard- oder Organolithium-Reagenzien und die Halogenierung und anschließende Hydrolyse von Methylketonen in der Haloform-Reaktion. EP1657230 offenbart ein Verfahren zur Herstellung von 3,5,5-Trimethylhexansäure durch Oxidation eines Aldehyds.

Die Hydrocarboxylierung von Olefinen ist eine sehr aussichtsreiche und umweltfreundliche Methode zum Erhalt von Carbonsäuren. Die Herstellung von Essigsäure erfolgt durch Carbonylierung von Methanol, die mit lodid durchgeführt wird.

Bei der Koch-Reaktion wird die Addition von Wasser und Kohlenmonoxid an Alkene durch starke Basen katalysiert. Diese Methode funktioniert gut bei Alkenen, die sekundäre und tertiäre Carbokationen bilden, z.B. Isobutylen zu Pivalinsäure. Die mit gleichzeitiger Addition von CO und H₂O an Alkene/Alkine verlaufende Hydrocarboxylierung stellt eine direkte und bequeme Methode zur Synthese von Carbonsäuren zur Verfügung.

Der Erfindung lag die Aufgabe zugrunde, ein Verfahren bereitzustellen, welches bei der Pd-katalysierten Hydroxycarbonylierung von Diisobuten (DIBN) einen guten Umsatz liefert. Die Umsetzung soll hierbei in einem Schritt erfolgen.

Gelöst wird die Aufgabe durch ein Verfahren nach Anspruch 1.

Verfahren umfassend die Verfahrensschritte:
a) Zugabe von Diisobuten,
b) Zugabe einer Verbindung, welche Pd umfasst, wobei das Pd in der Lage ist einen Komplex auszubilden,
c) Zugabe des Liganden **L1:** 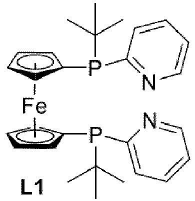 wobei der Ligand **L1** in einer Menge zugeben wird in dem Bereich von 1,0 mol bis 2,0 mol Ligand pro mol Pd,
d) Zugab von Essigsäure,
e) Zuführen von CO,
f) Erhitzen des Reaktionsgemisches, so dass das Diisobuten zur Verbindung **P1** umgesetzt wird:

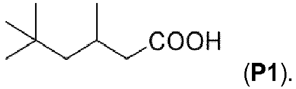

In einer Variante des Verfahrens ist die Verbindung im Verfahrensschritt b) ausgewählt aus: PdCl₂, PdBr₂, Pd(acac)₂, Pd(dba)₂ (dba = Dibenzylidenaceton), PdCl₂(CH₃CN)₂.

In einer Variante des Verfahrens ist die Verbindung im Verfahrensschritt b) Pd(acac)₂.

In einer Variante des Verfahrens wird der Ligand **L1** in einer Menge zugeben in dem Bereich von 1,25 mol bis 1,75 mol Ligand pro mol Pd.

In einer Variante des Verfahrens wird das Reaktionsgemisch im Verfahrensschritt f) auf eine Temperatur im Bereich von 80 °C bis 160 °C erhitzt.
Vorzugsweise auf eine Temperatur im Bereich von 100 °C bis 140 °C.

In einer Variante des Verfahrens erfolgt das Zuführen von CO im Verfahrensschritt e) so, dass die Reaktion bei einem CO-Druck im Bereich von 10 bar bis 40 bar verläuft. Vorzugsweise im Bereich von 10 bar bis 30 bar.

In einer Variante des Verfahrens umfasst dieses den zusätzlichen Verfahrensschritt g): g) Zugabe von Schwefelsäure.

Im Folgenden wird die Erfindung anhand von Ausführungsbeispielen näher erläutert.

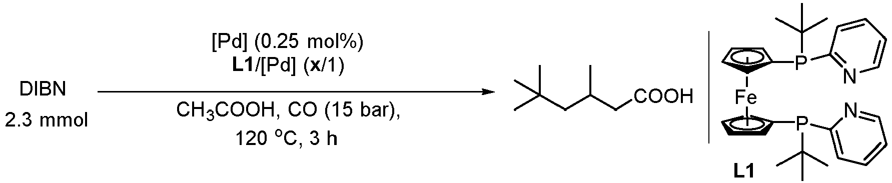

Ein 4-mL-Vial wurde mit [Pd(acac)₂] (1,75 mg, 0,25 mol-%), L1 (4,44 mg, 0,375 mol-%), H₂SO₄ (3,1 mg, 1,4 mol-%) und einem im Ofen getrockneten Rührstab beschickt. Dann wird das Vial mit Septen (PTFE-beschichteter Styrol-Butadien-Kautschuk) und Phenolharzdeckel verschlossen. Das Vial wird dreimal evakuiert und mit Argon wieder befüllt. Mit einer Spritze wurden H₂O (0,29 ml), Essigsäure (0,85 ml) und Diisobuten (DIBN) (2,3 mmol) in das Vial gegeben. Das Vial wurde in eine Legierungsplatte gestellt, die in einen Autoklaven (300 ml) der Reihe 4560 von Parr Instruments unter Argonatmosphäre überführt wurde. Nach dreimaligem Spülen des Autoklaven mit CO wurde der CO-Druck auf 15 bar bei Raumtemperatur erhöht, und anschließend mit N₂ auf einen Druck von 25 bar. Die Reaktion wurde 3 h bei 120 °C durchgeführt. Nach Beendigung der Reaktion wurde der Autoklav auf Raumtemperatur abgekühlt und vorsichtig entspannt. Dann wurde Isooctan (100 ul) als interner Standard zugegeben. Der Umsatz wurde durch GC-Analyse gemessen.

Der oben beschriebene Versuch wurde unter Variation des Verhältnisses **L1**/Pd wiederholt. Alle anderen Parameter wurden beibehalten.

Die Ergebnisse sind in der nachfolgenden Tabelle zusammengestellt.

| Eintrag | **L1**/Pd (mmol/mmol) - **L1** (x mol-%) | Umsatz (%) |
|---|---|---|
| 1* | 1.5/1 - 0,375 | 92 |
| 2* | 2/1 - 0,5 | 83 |
| 3 | 3/1 - 0,75 | 74 |
| 4 | 4/1 - 1,0 | 69 |

| | | |
|---|---|---|
| * erfindungsgemäßes Verfahren | | |

Wie die Versuchsergebnisse zeigen wird die Aufgabe durch das erfindungsgemäße Verfahren gelöst.

## Patentansprüche

1. Verfahren umfassend die Verfahrensschritte:
a) Zugabe von Diisobuten,
b) Zugabe einer Verbindung, welche Pd umfasst, wobei das Pd in der Lage ist einen Komplex auszubilden,
c) Zugabe des Liganden **L1:** 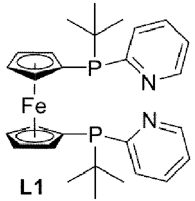 wobei der Ligand **L1** in einer Menge zugeben wird in dem Bereich von 1,0 mol bis 2,0 mol Ligand pro mol Pd,
d) Zugab von Essigsäure,
e) Zuführen von CO,
f) Erhitzen des Reaktionsgemisches, so dass das Diisobuten zur Verbindung **P1** umgesetzt wird:

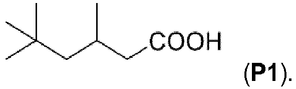

2. Verfahren nach Anspruch 1,
wobei die Verbindung im Verfahrensschritt b) ausgewählt ist aus: PdCl₂, PdBr₂, Pd(acac)₂, Pd(dba)₂ (dba = Dibenzylidenaceton), PdCl₂(CH₃CN)₂.

3. Verfahren nach einem der Ansprüche 1 bis 2,
wobei der Ligand **L1** in einer Menge zugeben wird in dem Bereich von 1,25 mol bis 1,75 mol Ligand pro mol Pd.

4. Verfahren nach einem der Verfahrensschritte 1 bis 3,
wobei das Reaktionsgemisch im Verfahrensschritt f) auf eine Temperatur im Bereich von 80 °C bis 160 °C erhitzt wird.

5. Verfahren nach einem der Verfahrensschritte 1 bis 4,
wobei das Zuführen von CO im Verfahrensschritt e) so erfolgt, dass die Reaktion bei einem CO-Druck im Bereich von 10 bar bis 40 bar verläuft.

6. Verfahren nach einem der Verfahrensschritte 1 bis 5,
wobei das Verfahren den zusätzlichen Verfahrensschritt g) umfasst: g) Zugabe von Schwefelsäure.

## Claims

1. Process comprising the process steps of:
a) addition of diisobutene,
b) addition of a compound comprising Pd, wherein the Pd is capable of forming a complex,
c) addition of the ligand **L1:** 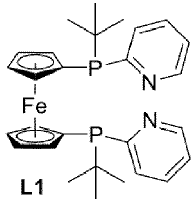 wherein the ligand **L1** is added in an amount in the range from 1.0 mol
to 2.0 mol of ligand per mole of Pd,
d) addition of acetic acid,
e) feeding in of CO,
f) heating of the reaction mixture such that the diisobutene is converted to the compound **P1:**

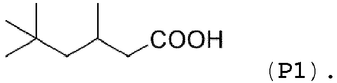

2. Process according to Claim 1,
wherein the compound in process step b) is selected from:
PdCl₂, PdBr₂, Pd(acac)₂, Pd(dba)₂ (dba = dibenzylideneacetone), PdCl₂(CH₃CN)₂.

3. Process according to any of Claims 1 to 2,
wherein the ligand **L1** is added in an amount in the range from 1.25 mol to 1.75 mol of ligand per mole of Pd.

4. Process according to any of process steps 1 to 3,
wherein the reaction mixture is heated to a temperature in the range from 80°C to 160°C in process step f).

5. Process according to any of process steps 1 to 4,
wherein the CO is fed in in process step e) such that the reaction proceeds under a CO pressure in the range from 10 bar to 40 bar.

6. Process according to any of process steps 1 to 5,
wherein the process comprises the additional process step g):
g) addition of sulfuric acid.

## Revendications

1. Procédé comprenant les étapes de procédé :
a) ajout de diisobutène,
b) ajout d'un composé, qui comprend du Pd, le Pd étant en l'état de former un complexe,
c) ajout du ligand L1 : 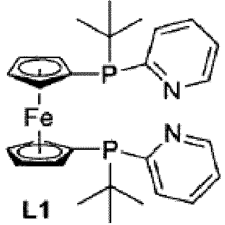 le ligand L1 étant ajouté en une quantité dans la plage de 1,0 mole à 2,0 moles de ligand par mole de Pd,
d) ajout d'acide acétique,
e) introduction de CO,
f) chauffage du mélange réactionnel, de sorte que le diisobutène est transformé en composé P1 :

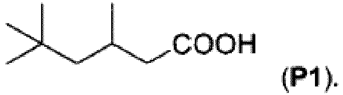

2. Procédé selon la revendication 1,
le composé dans l'étape de procédé b) étant choisi parmi :
PdCl₂, PdBr₂, Pd(acac)₂, Pd(dba)₂ (dba = dibenzylidèneacétone), PdCl₂(CH₃CN)₂.

3. Procédé selon l'une quelconque des revendications 1 et 2,
le ligand L1 étant ajouté en une quantité dans la plage de 1,25 mole à 1,75 mole de ligand par mole de Pd.

4. Procédé selon l'une quelconque des étapes de procédé 1 à 3,
le mélange réactionnel dans l'étape de procédé f) étant chauffé à une température dans la plage de 80 °C à 160 °C.

5. Procédé selon l'une quelconque des étapes de procédé 1 à 4,
l'introduction de CO dans l'étape de procédé e) étant réalisée de telle façon que la réaction se déroule à une pression de CO dans la plage de 10 bars à 40 bars.

6. Procédé selon l'une quelconque des étapes de procédé 1 à 5,
le procédé comprenant l'étape de procédé g) supplémentaire :
g) ajout d'acide sulfurique.
